(19) Europäisches Patentamt
European Patent Office
Office européen des brevets

(11) Publication number: 0 281 734 B1

# (12) EUROPEAN PATENT SPECIFICATION

(45) Date of publication of patent specification: **19.05.93**

(51) Int. Cl.$^5$: **C07K 7/10**, C12P 21/02, A61K 37/02, C12N 5/00

(21) Application number: **88100799.1**

(22) Date of filing: **20.01.88**

## (54) Polypeptide having an antibacterial activity, and its use.

(30) Priority: **23.01.87 JP 14806/87**

(43) Date of publication of application:
**14.09.88 Bulletin 88/37**

(45) Publication of the grant of the patent:
**19.05.93 Bulletin 93/20**

(84) Designated Contracting States:
**CH DE FR GB IT LI**

(56) References cited:

**THE JOURNAL OF BIOLOGICAL CHEMISTRY, vol. 260, no. 12, 25th June 1985, pages 7174-7177, The American Society of Biological Chemists, Inc., US; M. OKADA et al.: "Primary structure of sarcotoxin I, an antibacterial protein induced in the hemolymph of Sarcophaga peregrina (flesh fly) larvae"**

**BIOCHEMICAL JOURNAL, vol. 211, 1983, pages 727-734, GB; M. OKADA et al.: "Purification and characterization of an antibacterial protein from haemolymph of Sarcophaga peregrina (flesh-fly) larvae"**

**DRUGS OF THE FUTURE, vol. 13, no. 1, 1988, pages 59-68; S. NATORI: "Future drugs mimicking insect defense proteins: Sarcophaga lectin and sarcotoxin I"**

(73) Proprietor: **SANWA KAGAKU KENKYUSHO CO., LTD.**
**No. 35, Higashi-sotobori-cho**
**Higashi-ku Nagoya-shi Aichi-ken(JP)**

(72) Inventor: **Natori, Shunji**
**2208-19, Fukawa Tone-machi**
**Kita-soma-gun Ibaragi-ken(JP)**

(74) Representative: **Wächtershäuser, Günter, Dr.**
**Tal 29**
**W-8000 München 2 (DE)**

EP 0 281 734 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a novel biologically active polypeptide, method of obtaing same as well as use thereof. The polypeptide is obtained through a cultivation of and insect cell an more particularly a cell line established from flesh fly (Sarcophaga peregrina) embryo.

It has been known that a certain antibacterial substance will appear in a body fluid, when a vaccine is inoculated to an invertebrate such as insecta ["Eur. J. Biochem." Vol. 106, page 7 (1980)].

The present inventor has also found such a fact on the Sarcophaga peregrina that a certain antibacterial polypeptide appears in its body fluid, when a larva of the insect is injured in its body, the polypeptide being separated and purified to investigate its physicochemical properties [see Jap. Pats. 59 - 13730 (A) published January 24, 1984 and 61 - 122299 (A) published June 10, 1986].

Since the polypeptide produced by the insect shows a wide antibacterial spectrum, almost no toxicity and is one of proteins, the substance has been expected as one of edible antiobiotics but has difficulties in its productivities inclusive of insurance of the raw material (the insect per se), isolation and purification thereof.

A basic object of the invention, therefore, lies in providing a method of obtaining a biologically active polypeptide in a reasonable cost to allow an application thereof for industrial production.

According to the invention, the basic object can be attained by a method which comprises steps of cultivating a cell line designated as NIH Sape-4 and previously established from Sarcophaga peregrina embryo (see Develop. Growth and Diff. 23 (1980) 11-19), subjecting a resulting culture medium to an ion-exchange chromatography, a thermal treatment and a gel filtration chromatography to collect a physically active polypeptide through a separation and fractioning, and then subjecting a resulting active fraction to HPLC to purify the polypeptide through an adsorption, elution and fractioning.

The resulting polypeptide is novel one disclosed in no literature, has an amino acid sequence of

Gly-Trp-Ile-Arg-Asp-Phe-Gly-Lys-Arg-Ile-Glu-Arg-Val-Gly-Gln-His-
Thr-Arg-Asp-Ala-Thr-iLe-Gln-Thr-Ile-Ala-Val-Ala-Gln-Gln-Ala-Ala-
Asn-Val-Ala-Ala-Thr-Leu-Lys

and shows a wide antibacterial spectrum. The polypeptide sometimes has a further amino acid of glycine (Gly) at C-terminal end of the sequence.

Therefore, the present invention also intends to such novel polypeptide per se and use thereof as anti-bacterial agent.

According to the method of the invention, such useful polypeptide can be obtained in a large amount by merely enlarging a scale for the cultivation of the cell line.

The polypeptide according to the invention is excellent in thermal stability, as apparent from that the method comprises the thermal treatment, which means that it is useful as an antibacterial additive for foodstuffs and more particularly for those requiring a thermal processing.

The polypeptide shows almost no toxicity, similar to those as already obtained by the present inventor.

In case of preparing a drug with use of the polypeptide as an effective ingredient, there is no limitation in form thereof and thus the drug may be made into various forms for oral or none-oral adminstration. As a dosage for therapeutic purpose, it is preferable to give on the basis of the polypeptide in a range of 1 to 500mg/day, for instance 5mg/day for an adult.

The invention will now be further explained with reference to an Example for obtaining a biologically active polypeptide, Pharmacological Test Example as well as Medicine Preparation Example, which shall refer to drawings, wherein

Fig. 1 is a chromatogram showing an elution pattern on a biologically active polypeptide according to the invention, when Sephadex G-50 column is employed in after heat treating, to obtain the same;

Fig. 2 is a chromatogram similar to that of Fig. 1 but in the second stage using CM-cellulose column; and

Fig. 3 is a chromatogram similar to those in Figs. 1 and 2 but in the final stage of reverse phase HPLC.

Example

a) Cultivation of cell

In a sterilized culture medium proposed by Mitsuhasi et al (sodium chloride 7g/l, potassium chloride 0.2g/l, calcium chloride dihydrate 0.2g/l, magnesium chloride hexahydrate 0.1g/l, sodium hydrogen phosphate monohydrate 0.2g/l, sodium hydrogen carbonate 0.12g/l, glucose 4g/l. lactoalbuminhydrolysate 6.5g/l and yeastlate 5.0g/l; pH 6.5), cells established from Sarcophaga peregrina embryo (the cells being named as --NIH Sape-4 cell--) were inoculated in an amount of 1 x $10^6$ cells/ml of the culture medium, and cultivated at 25°C for 7 to 10 days. A passage was done, when the number of cells reached about 2 x $10^7$ cells/ml of the medium and the medium was recovered through a centrifugal treatment.

b) Separation and purification of an antibacterial polypeptide

In 500ml of the culture medium obtained by the procedure described in said Item a), 1500ml of 10mM-phosphate buffer (pH 6.0) were added to control the pH and salt concentration of the medium. The resulting solution was lead to a carboxymethylcellulose column (3.4 x 20.0cm) which was then washed with a fresh buffer solution same with the above.

To the column, 520mM-NaCl containing phosphate buffer was passed to collect each fraction by 5ml. An absorbance at 280nm, an antibacterial activity according to the method of OKADA et al and using an Escherichia coli (K12 594 strain) [ "Biochem. J." Vol. 211, pages 724 to 734 (1983)] as well as absorbance at 650nm were measured on each fraction to identify fraction(s) having antibacterial activity.

The antibacterial fractions were combined and heated to 100 °C for 10 minutes and then centrifugally treated to remove a precipitate formed therein. The resulting supernatant was concentrated through a ultrafiltration. The concentrate was treated with Sephadex G-50 column (1.5 x 60.0cm) and an elute was fractioned by each 2ml to determine an absorbance at 280nm and antibacterial activity, in the manner similar to the above. In this case, 130mM-NaCl containing phosphate buffer (pH 6.0) was employed to cause the elution. Results are shown in Fig. 1.

The antibacterial fractions apparent from the Figure were combined and diluted with use of 10mM-phosphate buffer to make its volume in 5 times. The resulting solution was subjected to carboxymethylcellulose column (2.0 x 4.0cm) and an adsorption was eluted with a linear gradient method using 25mM to 400mM-NaCl containing phosphate buffer. An absorbance at 280nm and antibacterial activity of the elute were measured as the above manner to obtain results shown in Fig. 2.

Among the antibacterial fractions 1 and 2 apparent from the Figure, the latter fractions were combined and fractioned with a reverse phase HPLC (Synchropack RPP-C18 column) to obtain results shown in Fig. 3.

The fractions corresponding to the main peak in the Figure were obtained to afford the desired biologically active polypeptide which was named as --KM-1--.

Followings are terms or conditions for the reverse phase HPLC.

| | |
|---|---|
| Reagent A : | 0.05% Trifluoroacetate/water, |
| Reagent B : | 0.05% Trifluoroacetate/99% Acetonitrile, |
| Gradient : | Linear gradient with use of 5% Reagent B in Reagent A and 43% Reagent B in Reagent A, |
| Flow velocity : | 2ml/min. |

c) Determination of amino acid sequence for KM-1

An amino acid sequence for KM-1 was checked with use of a gas-phase protein sequencer (Type 470A Protein Sequencer marketed by Applied Biosystems Inc.) to determine the same as follows.

Gly-Trp-Ile-Arg-Asp-Phe-Gly-Lys-Arg-Ile-Glu-Arg-Val-Gly-Gln-His-
Thr-Arg-Asp-Ala-Thr-iLe-Gln-Thr-Ile-Ala-Val-Ala-Gln-Gln-Ala-Ala-
Asn-Val-Ala-Ala-Thr-Leu-Lys-(Gly)

Sometimes Gly in the parenthesis was added to C-terminal end.

Pharmacological Test Example

(Measurement Test Example)

An antibacterial spectrum of KM-1 was measured in accordance with the method proposed by OKADA et al ["Biochem. J." Vo. 211, pages 727 to 734 (1983)] to obtain results shown in a following Table. As apparent from the Table, the KM-1 has relatively wide antibacterial spectrum.

| Kind of bacteria | MIC |
|---|---|
| Escherichia coli K-12 | 0.20 |
| Shigella sonnei JS11746 | 0.08 |
| Proteus vulgaris OX19 | 0.30 |
| Corynebacterium hovis 1810 | 0.30 |
| Bacillis subtilis APJ | 0.08 |

MIC : Minimum Inhibitory Concentration

Medicine Preparation Example (Dry powder for injection)

The biologically active polypeptide (KM-1) was sterilized in a conventional manner, aseptically charged the same into each vial by 5mg, freeze dried to make the same into a dry powder and sealed the vial.

When using for injection, the dry powder is dissolved into saline, aqua pro injection or the like.

## Claims

1. A biologically active polypeptide having an amino acid sequence shown by the formula of

Gly-Trp-Ile-Arg-Asp-Phe-Gly-Lys-Arg-Ile-Glu-Arg-Val-Gly-Gln-His-
Thr-Arg-Asp-Ala-Thr-iLe-Gln-Thr-Ile-Ala-Val-Ala-Gln-Gln-Ala-Ala-
Asn-Val-Ala-Ala-Thr-Leu-Lys.

2. A method of obtaining a biologically active polypeptide having an amino acid sequence shown by the formula of

Gly-Trp-Ile-Arg-Asp-Phe-Gly-Lys-Arg-Ile-Glu-Arg-Val-Gly-Gln-His-
Thr-Arg-Asp-Ala-Thr-iLe-Gln-Thr-Ile-Ala-Val-Ala-Gln-Gln-Ala-Ala-
Asn-Val-Ala-Ala-Thr-Leu-Lys,

which comprises steps of cultivating a cell line designated as NIH Sape-4 and established from Sarcophaga peregrina embryo, subjecting a resulting culture medium to an ion-exchange chromatography, a thermal treatment and a gel filtration chromatography to collect the biologically active polypeptide through a separation and fractioning, and then subjecting a resulting active fraction to HPLC to purify the polypeptide through an adsorption. elution and fractioning.

3. An antibacterial agent which comprises an effective amount of a biologically active polypeptide having an amino acid sequence shown by the formula of

Gly-Trp-Ile-Arg-Asp-Phe-Gly-Lys-Arg-Ile-Glu-Arg-Val-Gly-Gln-His-
Thr-Arg-Asp-Ala-Thr-iLe-Gln-Thr-Ile-Ala-Val-Ala-Gln-Gln-Ala-Ala-
Asn-Val-Ala-Ala-Thr-Leu-Lys,

and a pharmacologically acceptable inert carrier for the polypeptide.

**Patentansprüche**

1. Biologisch aktives Polypeptid mit einer Aminosäuresequenz, die durch die Formel

**Gly-Trp-Ile-Arg-Asp-Phe-Gly-Lys-Arg-Ile-Glu-Arg-Val-Gly-**
**Gln-His-Thr-Arg-Asp-Ala-Thr-Ile-Gln-Thr-Ile-Ala-Val-Ala-**
**Gln-Gln-Ala-Ala-Asn-Val-Ala-Ala-Thr-Leu-Lys**

dargestellt wird.

2. Verfahren zum Erhalten eines biologisch aktiven Polypeptids mit einer Sequenz, die durch die Formel

**Gly-Trp-Ile-Arg-Asp-Phe-Gly-Lys-Arg-Ile-Glu-Arg-Val-Gly-**
**Gln-His-Thr-Arg-Asp-Ala-Thr-Ile-Gln-Thr-Ile-Ala-Val-Ala-**
**Gln-Gln-Ala-Ala-Asn-Val-Ala-Ala-Thr-Leu-Lys**

dargestellt wird, umfassend die Stufen: Kultivieren einer aus Sarcophaga peregrina-Embryo angelegten, als NIH-Sape 4 bezeichneten Zellinie, Unterwerfen des Kulturmediums unter eine Ionenaustauschchromatographie, eine thermische Behandlung und eine Gelfiltrationschromatographie, um das biologisch aktive Polypeptid durch Abtrennung und Fraktionierung zu gewinnen, und anschließendes Unterwerfen einer resultierenden aktiven Fraktion unter HPLC, um das Polypeptid durch Adsorption, Eluieren und Fraktionieren zu reinigen.

3. Antibakterielles Mittel, umfassend eine wirksame Menge eines biologisch aktiven Polypeptids mit einer Aminosäuresequenz, die durch die Formel

**Gly-Trp-Ile-Arg-Asp-Phe-Gly-Lys-Arg-Ile-Glu-Arg-Val-Gly-**

**Gln-His-Thr-Arg-Asp-Ala-Thr-Ile-Gln-Thr-Ile-Ala-Val-Ala-**
**Gln-Gln-Ala-Ala-Asn-Val-Ala-Ala-Thr-Leu-Lys**

dargestellt wird,
und einen pharmakologisch annehmbaren inerten Träger für das Polypeptid.

## Revendications

1. Polypeptide biologiquement actif ayant une séquence d'acides aminés représentée par la formule suivante

   Gly-Trp-Ile-Arg-Asp-Phe-Gly-Lys-Arg-Ile-Glu-Arg-Val-Gly-Gln-His-
   Thr-Arg-Asp-Ala-Thr-iLe-Gln-Thr-Ile-Ala-Val-Ala-Gln-Gln-Ala-Ala-
   Asn-Val-Ala-Ala-Thr-Leu-Lys.

2. Procédé de préparation d'un polypeptide biologiquement actif ayant une séquence d'acides aminés représentée par la formule suivante :

   Gly-Trp-Ile-Arg-Asp-Phe-Gly-Lys-Arg-Ile-Glu-Arg-Val-Gly-Gln-His-
   Thr-Arg-Asp-Ala-Thr-iLe-Gln-Thr-Ile-Ala-Val-Ala-Gln-Gln-Ala-Ala-
   Asn-Val-Ala-Ala-Thr-Leu-Lys,

   qui comprend les étapes consistant à cultiver une lignée de cellules désignée sous le nom de NIH Sape −4 et établie à partir de l'embryon de SARCOPHARGA PEREGRINA, soumettre un milieu de culture obtenu à une chromatographie par échange d'ions, à un traitement thermique et à une chromatographie de filtration sur gel afin de collecter le polypeptide biologiquement actif par séparation et fractionnement, puis soumettre une fraction active obtenue à une HPLC afin de purifier le polypeptide par adsorption, élution et fractionnement.

3. Agent antibactérien comprenant une quantité efficace d'un polypeptide biologiquement actif ayant une séquence d'acides aminés représentée par la formule

   Gly-Trp-Ile-Arg-Asp-Phe-Gly-Lys-Arg-Ile-Glu-Arg-Val-Gly-Gln-His-
   Thr-Arg-Asp-Ala-Thr-iLe-Gln-Thr-Ile-Ala-Val-Ala-Gln-Gln-Ala-Ala-
   Asn-Val-Ala-Ala-Thr-Leu-Lys,

   et un support inerte pharmacologiquement acceptable pour le polypeptide.

FIG. 1

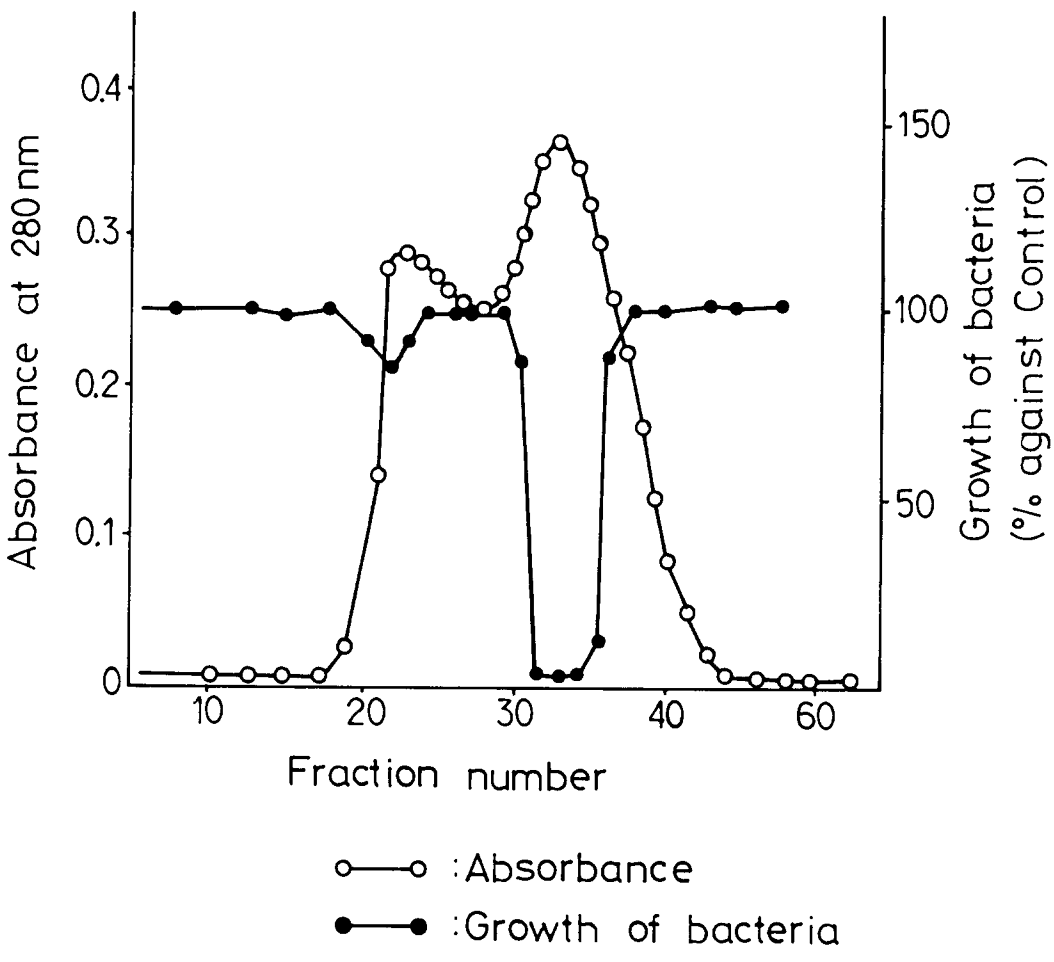
0.4
0.3
0.2
0.1
0
Absorbance at 280nm
150
100
50
Growth of bacteria
(% against Control)
10
20
30
40
60
Fraction number
:Absorbance
:Growth of bacteria

FIG. 2

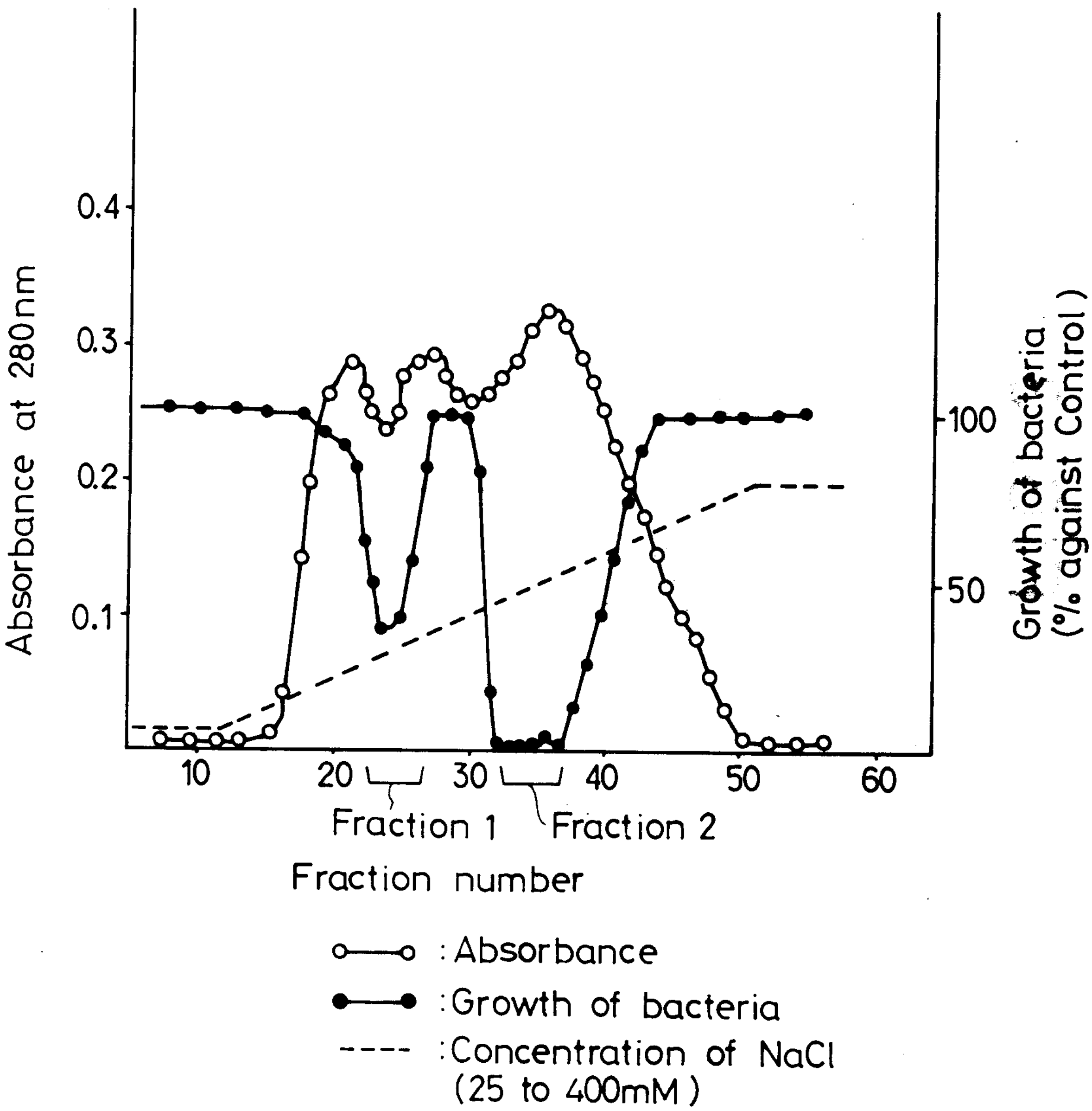
0.4
0.3
0.2
0.1
Absorbance at 280nm
100
50
Growth of bacteria
(% against Control)
10
20
30
40
50
60
Fraction 1
Fraction 2
Fraction number
: Absorbance
: Growth of bacteria
: Concentration of NaCl
(25 to 400mM)

FIG. 3

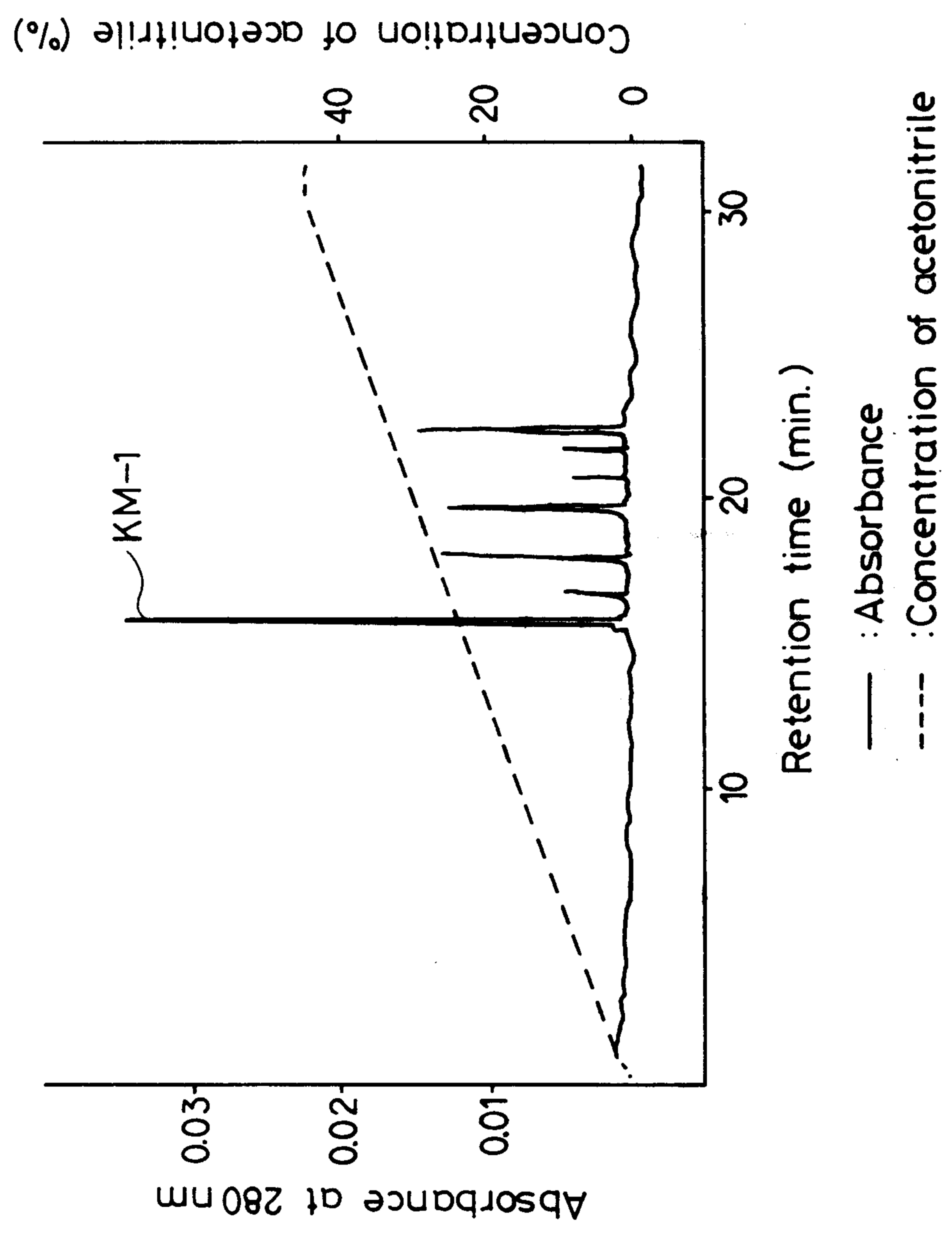
Absorbance at 280 nm
0.03
0.02
0.01
Concentration of acetonitrile (%)
40
20
0
KM-1
10
20
30
Retention time (min.)
: Absorbance
:Concentration of acetonitrile